# EUROPEAN PATENT SPECIFICATION

(11) **EP 4 282 373 B1**
(45) Date of publication and mention of the grant of the patent: **04.09.2024**
(21) Application number: 23174838.5
(22) Date of filing: 23.05.2023
(51) Int. Cl.: A61C 7/00, A61C 7/12, A61C 7/14, A61C 7/20

(54) **CORRECTION IMAGE GENERATION METHOD FOR ORTHODONTIC TREATMENT AND APPARATUS FOR PERFORMING THE METHOD**
KORREKTURBILDERZEUGUNGSVERFAHREN FÜR DIE ORTHODONTISCHE BEHANDLUNG UND VORRICHTUNG ZUR DURCHFÜHRUNG DES VERFAHRENS
PROCÉDÉ DE GÉNÉRATION D'IMAGE DE CORRECTION POUR TRAITEMENT ORTHODONTIQUE ET APPAREIL POUR LA MISE EN OEUVRE DU PROCÉDÉ

(30) Priority: 23.05.2022 KR 20220063093
(43) Date of publication of application: 29.11.2023
(73) Proprietor: Yoat Corporation, Seoul 08513 (KR)
(72) Inventor: JUNG, Youn Ho, 08513 Seoul (KR)
(74) Representative: Isarpatent

(56) References cited:
- WO-A2-01/80761
- US-A1- 2019 015 177
- US-A1- 2020 281 702

## Description

### BACKGROUND

### 1. Field

Embodiments of the present disclosure relate to a correction image generation technique for orthodontic treatment.

### 2. Discussion of Related Art

Dental treatment is a series of treatments for preventing, diagnosing, and treating diseases or abnormal conditions in a facial region including the teeth and surrounding tissues and the oral cavity.

Recently, many orthodontic treatments have been performed in addition to treatments necessary to improve oral hygiene and to maintain healthy teeth. Orthodontic treatment involves not only simply straightening crooked teeth, but also creating healthy oral tissues and beautiful facial features by correcting various skeletal inconsistencies that may occur during growth so that they can function normally.

A method in which orthodontic treatment is performed using a wire after an orthodontic bracket is mounted on teeth is widely used as an orthodontic treatment method. In this orthodontic treatment method, the wire is replaced according to an orthodontic treatment process.

However, when an oral cavity is scanned using an intraoral 3D scanner, the orthodontic bracket mounted on the teeth during orthodontic treatment is not accurately scanned. That is, there is a problem in that it is difficult to accurately check a position of a groove through which a wire is inserted into an orthodontic bracket in an image scanned using an intraoral 3D scanner.

Therefore, when a scanned image is used, there is a problem in that it is not possible to accurately bend the wire for insertion into the orthodontic bracket.

US2020/281702 discloses a device and method of generating a virtual 3D model of a dental site wherein scan data comprising a plurality of images of a dental site is received during an intraoral scan.

### SUMMARY

The invention is as defined in the appended claims.

Embodiments of the present disclosure are intended to correct an image by matching a pre-stored orthodontic bracket image with scan data obtained using an intraoral 3D scanner.

According to an aspect of the present disclosure, there is provided a computing device which includes one or more processors, and a memory configured to store one or more programs executed by the one or more processors, including an image acquisition part configured to acquire an oral image of an inside of a target patient's oral cavity using an intraoral 3D scanner, an image analyzer configured to generate a tooth image and an image of a bracket attached to a plurality of teeth through the obtained oral image, and an image corrector configured to match the image of the attached bracket and an orthodontic bracket image included in pre-stored orthodontic bracket information and to generate a correction image for the oral image based on the tooth image and the matching orthodontic bracket image.

The computing device may further include a provider configured to provide a user with the correction image generated by the image corrector.

The provider may provide bracket groove position information by calculating a position of a bracket groove in the correction image based on the matching orthodontic bracket information.

The image analyzer may generate an image of each tooth in the oral cavity based on the oral image, may generate an image of a bracket attached to each tooth, and may generate position information of the image of the bracket attached to each tooth based on an adhesive surface in the image of the attached bracket.

The image corrector may compare the image of the attached bracket with the pre-stored orthodontic bracket image and may extract an orthodontic bracket image that matches the image of the attached bracket in the pre-stored orthodontic bracket image.

The image corrector may place the matching orthodontic bracket image in the same portion as the image of the bracket attached to each tooth on the basis of the position information of the image of the attached bracket.

The image corrector may match the matching orthodontic bracket image and the image of the attached bracket using an iterative closest point (ICP) algorithm.

According to another aspect of the present disclosure, there is provided a correction image generation method for orthodontic treatment which is performed in a computing device including one or more processors, and a memory for storing one or more programs executed by the one or more processors, including obtaining an oral image of an inside of a target patient's oral cavity using an intraoral 3D scanner, generating a tooth image and an image of a bracket attached to a plurality of teeth through the obtained oral image, and matching the image of the attached bracket and an orthodontic bracket image included in pre-stored orthodontic bracket information and generating a correction image for the oral image based on the tooth image and the matching orthodontic bracket image.

The correction image generation method may further include providing the correction image generated by an image corrector to a user.

The providing of the correction image to the user may further include providing bracket groove position information by calculating a position of a bracket groove in the correction image based on the matching orthodontic bracket information.

The generating of the tooth image and the image of the bracket attached to the plurality of teeth may further include generating an image of each tooth in the oral cavity based on the oral image, generating an image of a bracket attached to each of the teeth, and generating position information of the image of the bracket attached to each tooth based on an adhesive surface in the image of the attached bracket.

The generating of the correction image may further include comparing the image of the attached bracket with the pre-stored orthodontic bracket image, and extracting an orthodontic bracket image that matches the image of the attached bracket in the pre-stored orthodontic bracket image.

The generating of the correction image may further include placing the matching orthodontic bracket image in the same portion as the image of the bracket attached to each tooth on the basis of the position information of the image of the attached bracket.

According to embodiments of the present disclosure, it is possible to obtain an accurate position of a bracket groove from scan data by matching the scan data obtained using an intraoral 3D scanner and a pre-stored orthodontic bracket image to correct an image.

### BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:
FIG. 1 is a block diagram illustrating a correction image generation apparatus for orthodontic treatment according to an embodiment of the present disclosure;
FIG. 2 is a view for describing a method of adjusting a position of a virtual 3D tooth model according to an embodiment of the present disclosure;
FIGS. 3 and 4 are diagrams illustrating a process of generating a correction image according to an embodiment of the present disclosure;
FIG. 5 is a flowchart illustrating a correction image generation method for orthodontic treatment according to an embodiment of the present disclosure; and
FIG. 6 is a block diagram illustrating and describing a computing environment including a computing device suitable for use in exemplary embodiments.

### DETAILED DESCRIPTION OF EXEMPLARY EMBODIMENTS

Hereinafter, specific embodiments of the present disclosure will be described in detail with reference to the accompanying drawings. The following detailed description is provided to assist the reader in gaining a comprehensive understanding of the methods, apparatuses, and/or systems described herein. However, the description is only exemplary, and the present disclosure is not limited thereto.

In describing embodiments of the present disclosure, when it is determined that detailed description of known techniques associated with the present disclosure would unnecessarily obscure the subject matter of the present disclosure, the detailed description thereof will be omitted. Also, terms used herein are defined in consideration of the functions of the present disclosure, and may be changed depending on the intent or custom of a user or operator. Accordingly, the terms should be defined based on the following overall description of this specification. The terminology used herein is only for the purpose of describing embodiments of the present disclosure and is not restrictive. The singular forms "a" and "an" are intended to include the plural forms as well, unless the context clearly indicates otherwise. It should be understood that the terms "comprises," "comprising," "includes," and/or "including" specify the presence of stated features, integers, steps, operations, elements, and/or components when used herein, but do not preclude the presence or addition of one or more other features, integers, steps, operations, elements, components, and/or groups thereof.

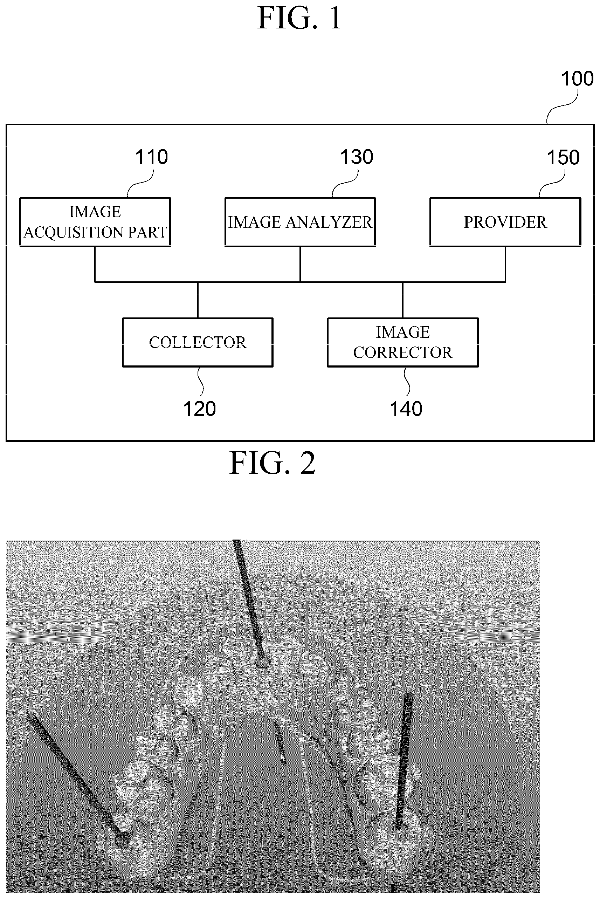

FIG. 1 is a block diagram illustrating a correction image generation apparatus for orthodontic treatment according to an embodiment of the present disclosure.

As illustrated in FIG. 1, the correction image generation apparatus 100 for orthodontic treatment according to the embodiment of the present disclosure may include an image acquisition part 110, a collector 120, an image analyzer 130, an image corrector 140, and a provider 150.

The image acquisition part 110 may acquire an oral image (scan data) of the inside of an oral cavity of a target patient using an intraoral 3D scanner. Specifically, the image acquisition part 110 may acquire an oral image of the target patient by inserting the intraoral 3D scanner into the oral cavity of the target patient and scanning the inside of the oral cavity. At this time, data acquired by the intraoral 3D scanner may be acquired by a computer and peripheral devices connected to the computer and then may be recorded in a memory device, for example, may have a structure that is generated and stored as a 3D stereo-lithography (STL) file. Meanwhile, in the present disclosure, the intraoral 3D scanner is used as a 3D scanning device, but the present disclosure is not limited thereto, and various scanning devices such as a coordinate measuring machine (CMM), a laser scanner, and an optical scanner may be used.

The collector 120 may receive orthodontic bracket information from an external server (for example, a server of a bracket manufacturer). Here, the orthodontic bracket information may include information on images, materials, bracket grooves, and the like of the orthodontic bracket for each manufacturer. Meanwhile, in the present disclosure, although the orthodontic bracket information is received from the external server, the present disclosure is not limited thereto, and the orthodontic bracket information may be stored in advance. Here, the orthodontic bracket is a device attached to a tooth, may be firmly attached to a surface of the orthodontic target tooth for orthodontic treatment, and may be interconnected using a wire. The orthodontic bracket may be formed with a bracket groove to be coupled with the wire. Thus, the orthodontic target tooth can be gradually moved by adjusting tension applied to the wire and adjusting a direction and magnitude of a force applied to the wire. Thus, a position and posture of the orthodontic target tooth are changed by the tension of the wire, and the orthodontic treatment is performed while the tooth is moved little by little.

The image analyzer 130 may analyze the oral image (the scan data) obtained from the image acquisition part 110 and may generate a tooth image and an image of a bracket attached to a plurality of teeth. Here, the images may be 3D images obtained from the scan data, for example, a 3D mesh image or a 3D model. For example, as illustrated in FIG. 2, the scan data generated and stored as an STL file is loaded by a computer and forms a virtual 3D dental model, and the virtual 3D dental model may be generated for each of an upper jaw and a lower jaw of the plurality of teeth. The upper jaw and lower jaw of which the virtual 3D dental model is formed may form any three axes with respect to the virtual 3D dental model, and a user may move and rotate the axes to arrange the upper jaw and the lower jaw in positions desired by the user.

In an exemplary embodiment, the image analyzer 130 may extract each of the tooth images based on the oral image and may extract the image of the bracket attached to each tooth. Also, the image analyzer 130 may generate position information of the attached brackets attached to each tooth based on an adhesive surface of the attached brackets (the brackets attached to the teeth). Here, the position information of the attached bracket may include an attachment surface of the tooth to which the attachment surface of the bracket is attached, and position coordinates of the bracket attached to the attachment surface of the tooth.

The image corrector 140 may generate a correction image based on the position information of the orthodontic bracket image, the tooth image, and the position information of the attached bracket that match the attached bracket image among the pre-stored orthodontic bracket images. Here, each of the tooth images may be changed by adjusting the left side, the right side, the front side, the back side, and a height according to necessity (progress of the orthodontic treatment).

In an exemplary embodiment, the image corrector 140 may compare a pre-stored orthodontic bracket image with an attached bracket image and may extract an orthodontic bracket image that matches the attached bracket image in the pre-stored orthodontic bracket information. In addition, the image corrector 140 may generate a correction image by combining the matching orthodontic bracket image based on the position information of the attached bracket with each of the tooth images. Here, the position information of the attached bracket may include position coordinates of the attachment surface of the tooth to which the attachment surface of the bracket is attached and the bracket attached to the attachment surface of the tooth. That is, the image corrector 140 may place the matching orthodontic bracket image based on the position information of the attached bracket in the same portion as the attached bracket image of each of the tooth images. At this time, the image corrector 140 may minimize a distance error between feature points of the matching orthodontic bracket image and feature points of the attached bracket image in order to match the matching orthodontic bracket image and the attached bracket image placed in the same portion in each of the tooth images. For example, the image corrector 140 may calculate a translation matrix and a rotation matrix using an iterative closest point (ICP) algorithm so that the distance error between feature points of the orthodontic bracket image and feature points of the corresponding attached bracket image can be minimized. In addition, the image corrector 140 may apply the calculated translation matrix and rotation matrix to the feature points of the matching orthodontic bracket image to match the attached bracket image and the matching orthodontic bracket image.

That is, due to the matching orthodontic bracket image based on the position information of the attached bracket being located in the same portion as the attached bracket image of each of the tooth images and the matching orthodontic bracket image and the attached bracket image matching each other using the ICP algorithm, accuracy of the ICP algorithm can be increased by giving an appropriate initial position of the two images (that is, a high degree of overlap between the matching orthodontic bracket image and the attached bracket image of the tooth image). Accordingly, the image corrector 140 may change an existing attached bracket image to the matching orthodontic bracket image.

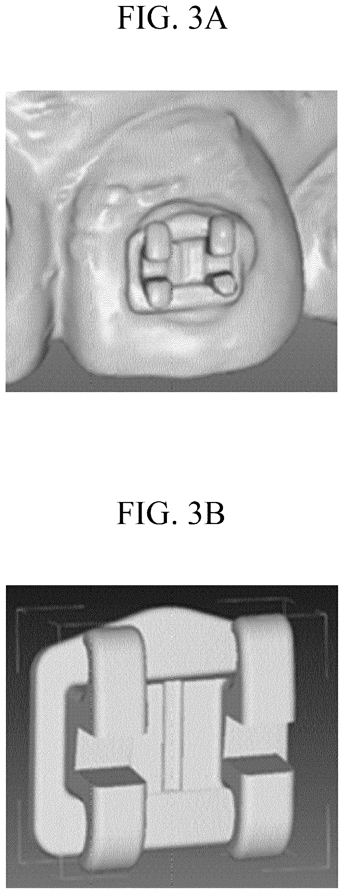

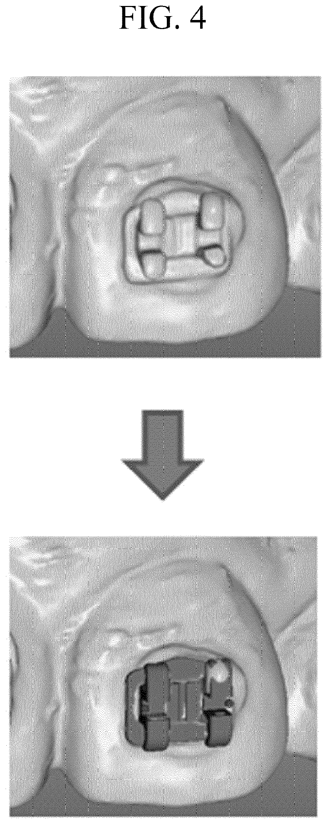

For example, as shown in FIG. 3, FIG. 3A is an attached bracket image obtained from an oral image, and FIG. 3B is a pre-stored orthodontic bracket image. Since it is difficult to accurately identify a position of the bracket groove of the orthodontic bracket in the oral image, it is possible to extract the orthodontic bracket image that matches the attached bracket image in the pre-stored orthodontic bracket information. In addition, as shown in FIG. 4, it is possible to change the attached bracket image on the tooth to the matching orthodontic bracket image using the matching orthodontic bracket image. Thus, it is possible to obtain the accurate position of the bracket groove from the oral image.

The provider 150 may provide a correction image generated by the image corrector 140. In addition, the provider 150 may calculate the position of the bracket groove in the correction image based on bracket groove information of the matching orthodontic bracket. In addition, the provider 150 may provide the bracket groove position information of each of the tooth images. That is, the provider 150 may generate a correction image for the oral image by changing the attached bracket image to a pre-stored orthodontic bracket image in the oral image for the inside of the oral cavity of the target patient.

Accordingly, the correction image generation apparatus 100 for orthodontic treatment according to the embodiment of the present disclosure may obtain an accurate position of the bracket groove from the scan data by matching the pre-stored orthodontic bracket and the scan data obtained using the intraoral 3D scanner and correcting the image.

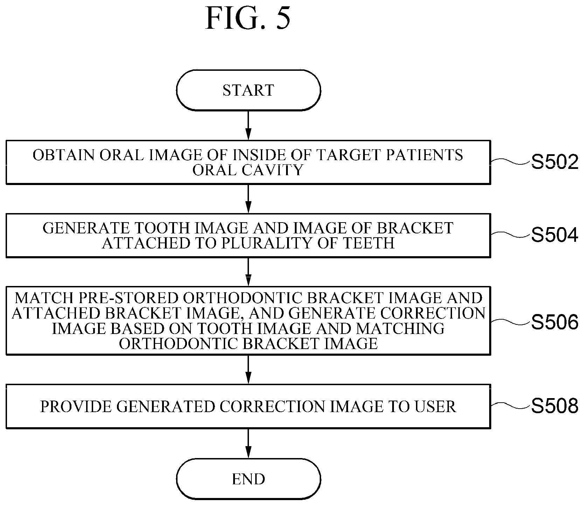

FIG. 5 is a flowchart for describing a correction image generation method for orthodontic treatment according to an embodiment of the present disclosure. The correction image generation method for orthodontic treatment according to the embodiment of the present disclosure may be performed in a computing device 12 including one or more processors and one or more memories that store one or more programs executed by the one or more processors. To this end, the correction image generation method for orthodontic treatment may be implemented in the form of a program or software including one or more computer-executable instructions and may be stored in the memory.

In addition, in the illustrated flowchart, although the method has been described by dividing the method into a plurality of steps, at least some of the steps may be performed in a reversed order, may be performed together in combination with other steps, may be omitted, may be divided into detailed steps, or may be performed by adding one or more steps that are not shown.

First, the computing device 12 obtains the scan data (the oral image) of the inside of a target patient's oral cavity using an intraoral 3D scanner (S502). Specifically, the computing device 12 may acquire the oral image of the target patient by inserting the intraoral 3D scanner into the oral cavity of the target patient and scanning the inside of the oral cavity.

Next, the computing device 12 analyzes the oral image to generate a tooth image and an image of a bracket attached to a plurality of teeth (S504). Here, the image may be a 3D image obtained from the scan data (the oral image). Specifically, the computing device 12 may generate an image of each tooth based on the oral image and may generate the image of the bracket attached to each tooth. Also, the computing device 12 may generate position information of the attached bracket attached to each tooth based on the adhesive surface of each of the attached brackets (each of the brackets attached to the teeth). Here, the position information of the attached bracket may include position coordinates of the attachment surface of the tooth to which the attachment surface of the bracket is attached, and position coordinates of the bracket attached to the attachment surface of the tooth.

Then, the computing device 12 matches the pre-stored orthodontic bracket image and the attached bracket image and generates a correction image based on the tooth image, the matching orthodontic bracket image, and the position information of the attached bracket (S506). Specifically, the computing device 12 may compare the pre-stored orthodontic bracket image with the attached bracket image and may extract an orthodontic bracket image that matches the attached bracket image in the pre-stored orthodontic bracket information. In addition, the computing device 12 may generate a correction image by combining the matching orthodontic bracket image based on the position information of the attached bracket with each of the tooth images.

Finally, the computing device 12 provides the generated correction image to the user (S508). At this time, the computing device 12 may provide bracket groove position information by calculating a position of the bracket groove in the correction image based on the bracket groove information of the matching orthodontic bracket.

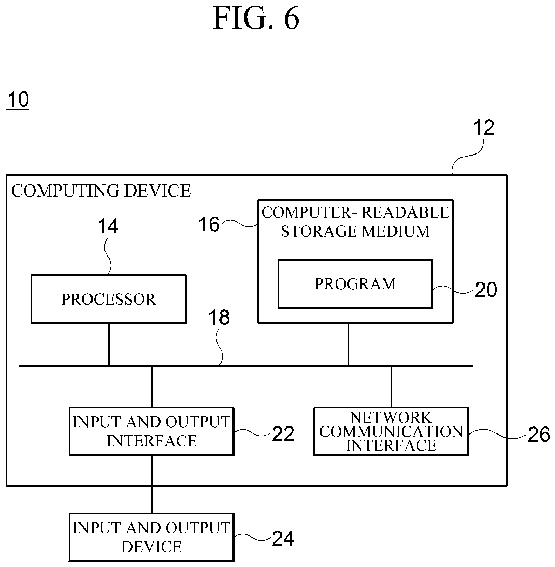

FIG. 6 is a block diagram illustrating and describing a computing environment including a computing device suitable for use in exemplary embodiments. In the illustrated embodiment, each component may have different functions and capabilities other than those which will be described below and may include additional components in addition to those which will be described below.

The illustrated computing environment 10 includes the computing device 12. In one embodiment, the computing device 12 may be a device for generating a correction image for orthodontic treatment.

The computing device 12 includes at least one processor 14, a computer-readable storage medium 16, and a communication bus 18. The processor 14 may be operated by the computing device 12 in accordance with the exemplary embodiments discussed above. For example, the processor 14 may execute one or more programs stored in the computer-readable storage medium 16. The one or more programs may include one or more computer-executable instructions, and the computer-executable instructions may be configured so that the computing device 12 performs operations in accordance with the exemplary embodiment when the instructions are executed by the processor 14.

The computer-readable storage medium 16 is configured to store computer-executable instructions or program codes, program data, and/or other suitable types of information. The program 20 stored in the computer-readable storage medium 16 includes a set of instructions executable by the processor 14. In one embodiment, the computer-readable storage medium 16 may be a memory (a volatile memory such as a random access memory, a non-volatile memory, or a suitable combination thereof), one or more magnetic disk storage devices, optical disc storage devices, flash memory devices, other types of storage media that can be accessed by the computing device 12 and can store desired information, or a suitable combination thereof.

The communication bus 18 interconnects various other components of the computing device 12 including the processor 14 and the computer-readable storage medium 16.

The computing device 12 may also include one or more input and output interfaces 22 that provide interfaces for one or more input and output devices 24, and one or more network communication interfaces 26. The input and output interfaces 22 and the network communication interfaces 26 are connected to the communication bus 18. The input and output devices 24 may be connected to other components of the computing device 12 via the input and output interfaces 22. The exemplary input and output device 24 may include a pointing device (such as a mouse or track pad), a keyboard, a touch input device (such as a touch pad or touch screen), a voice or sound input device, various types of input devices such as sensor devices and/or imaging devices, and/or output devices such as display devices, printers, speakers, and/or network cards. The exemplary input and output device 24 may be included in the computing device 12 as a component constituting the computing device 12 and may be connected to the computing device 12 as a separate device distinct from the computing device 12.

Although example embodiments of the present disclosure have been described in detail, it should be understood by those skilled in the art that various changes may be made without departing from the scope of the present disclosure. Therefore, the scope of the present disclosure is to be determined by the following claims and is not restricted or limited by the foregoing detailed description.

## Claims

1. A computing device (12) which includes one or more processors (14), and a memory configured to store one or more programs executed by the one or more processors, the computing device (12) comprising:
an image acquisition part (110) configured to acquire an oral image of an inside of a target patient's oral cavity using an intraoral 3D scanner;
an image analyzer (130) configured to generate a tooth image and an image of a bracket attached to a plurality of teeth through the obtained oral image; and
an image corrector (140) configured to match the image of the attached bracket and an orthodontic bracket image included in pre-stored orthodontic bracket information and to generate a correction image for the oral image based on the tooth image and the matching orthodontic bracket image.

2. The computing device of claim 1, further comprising a provider configured to provide a user with the correction image generated by the image corrector.

3. The computing device of claim 2, wherein the provider provides bracket groove position information by calculating a position of a bracket groove in the correction image based on the matching orthodontic bracket information.

4. The computing device of claim 1, wherein the image analyzer generates an image of each tooth in the oral cavity based on the oral image, generates an image of a bracket attached to each tooth, and generates position information of the image of the bracket attached to each tooth based on an adhesive surface in the image of the attached bracket.

5. The computing device of claim 4, wherein the image corrector compares the image of the attached bracket with the pre-stored orthodontic bracket image and extracts an orthodontic bracket image that matches the image of the attached bracket in the pre-stored orthodontic bracket image.

6. The computing device of claim 5, wherein the image corrector places the matching orthodontic bracket image in the same portion as the image of the bracket attached to each tooth on the basis of the position information of the image of the attached bracket.

7. The computing device of claim 6, wherein the image corrector matches the matching orthodontic bracket image and the image of the attached bracket using an iterative closest point (ICP) algorithm.

8. A correction image generation method for orthodontic treatment which is performed in a computing device (12) including one or more processors (14), and a memory for storing one or more programs executed by the one or more processors, the method comprising:
obtaining (S502) an oral image of an inside of a target patient's oral cavity using an intraoral 3D scanner;
generating (S504) a tooth image and an image of a bracket attached to a plurality of teeth through the obtained oral image; and
matching (S506) the image of the attached bracket and an orthodontic bracket image included in pre-stored orthodontic bracket information and generating a correction image for the oral image based on the tooth image and the matching orthodontic bracket image.

9. The correction image generation method of claim 8, further comprising providing the correction image generated by an image corrector to a user.

10. The correction image generation method of claim 9, wherein the providing of the correction image to the user further includes providing bracket groove position information by calculating a position of a bracket groove in the correction image based on the matching orthodontic bracket information.

11. The correction image generation method of claim 8, wherein the generating of the tooth image and the image of the bracket attached to the plurality of teeth further includes:
generating an image of each tooth in the oral cavity based on the oral image;
generating an image of a bracket attached to each of the teeth; and
generating position information of the image of the bracket attached to each tooth based on an adhesive surface in the image of the attached bracket.

12. The correction image generation method of claim 11, wherein the generating of the correction image further includes:
comparing the image of the attached bracket with the pre-stored orthodontic bracket image; and
extracting an orthodontic bracket image that matches the image of the attached bracket in the pre-stored orthodontic bracket image.

13. The correction image generation method of claim 12, wherein the generating of the correction image further includes placing the matching orthodontic bracket image in the same portion as the image of the bracket attached to each tooth on the basis of the position information of the image of the attached bracket.

## Patentansprüche

1. Computervorrichtung (12), aufweisend einen oder mehrere Prozessoren (14) und einen Speicher, der dafür eingerichtet ist, ein oder mehrere Programme zu speichern, die durch den einen oder die mehreren Prozessoren ausgeführt werden, wobei die Computervorrichtung (12) Folgendes umfasst:
einen Bildaufnahmeteil (110), der dafür eingerichtet ist, ein orales Bild eines Inneren der Mundhöhle eines Zielpatienten unter Verwendung eines intraoralen 3D-Scanners aufzunehmen;
einen Bildanalysator (130), der dafür eingerichtet ist, ein Zahnbild und ein Bild eines an mehreren Zähnen angebrachten Halteteils durch das erhaltene Mundbild zu erzeugen; und
eine Bildkorrekturvorrichtung (140), die dafür eingerichtet ist, das Bild des angebrachten Halteteils und ein orthodontisches Halteteilbild, das in vorab gespeicherten orthodontischen Halteteilinformationen enthalten ist,
abzugleichen und ein Korrekturbild für das orale Bild auf der Grundlage des Zahnbildes und des übereinstimmenden orthodontischen Halteteilbildes zu erzeugen.

2. Computervorrichtung nach Anspruch 1, umfassend des Weiteren eine Bereitstellungsvorrichtung, die dafür eingerichtet ist, einem Benutzer das durch die Bildkorrekturvorrichtung erzeugte Korrekturbild bereitzustellen.

3. Computervorrichtung nach Anspruch 2, wobei die Bereitstellungsvorrichtung Halteteilnutpositionsinformationen bereitstellt, indem sie eine Position einer Halteteilnut in dem Korrekturbild auf der Grundlage der übereinstimmenden orthodontischen Halteteilinformationen berechnet.

4. Computervorrichtung nach Anspruch 1, wobei der Bildanalysator ein Bild jedes Zahns in der Mundhöhle auf der Grundlage des oralen Bildes erzeugt, ein Bild eines an jedem Zahn angebrachten Halteteils erzeugt und Positionsinformationen des Bildes des an jedem Zahn angebrachten Halteteils auf der Grundlage einer Klebefläche in dem Bild des angebrachten Halteteils erzeugt.

5. Computervorrichtung nach Anspruch 4, wobei die Bildkorrekturvorrichtung das Bild des angebrachten Halteteils mit dem vorab gespeicherten orthodontischen Halteteilbild vergleicht und ein orthodontisches Halteteilbild extrahiert, das mit dem Bild des angebrachten Halteteils in dem vorab gespeicherten orthodontischen Halteteilbild übereinstimmt.

6. Computervorrichtung nach Anspruch 5, wobei die Bildkorrekturvorrichtung das übereinstimmende orthodontische Halteteilbild auf der Grundlage der Positionsinformationen des Bildes des angebrachten Halteteils im selben Abschnitt wie das Bild des an jedem Zahn angebrachten Halteteils platziert.

7. Computervorrichtung nach Anspruch 6, wobei die Bildkorrekturvorrichtung das übereinstimmende orthodontische Halteteilbild und das Bild des angebrachten Halteteils unter Verwendung eines Iterative Closest Point (ICP)-Algorithmus abgleicht.

8. Korrekturbilderzeugungsverfahren für eine orthodontische Behandlung, durchgeführt in einer Computervorrichtung (12) durchgeführt, die einen oder mehrere Prozessoren (14) und einen Speicher zum Speichern eines oder mehrerer Programme aufweist, die durch den einen oder die mehreren Prozessoren ausgeführt werden, wobei das Verfahren umfasst:
Erhalten (S502) eines oralen Bildes eines Inneren der Mundhöhle eines Zielpatienten unter Verwendung eines intraoralen 3D-Scanners;
Erzeugen (S504) eines Zahnbildes und eines Bildes eines an mehreren Zähnen angebrachten Halteteils durch das erhaltene Mundbild; und
Abgleichen (S506) des Bildes des angebrachten Halteteils und eines orthodontischen Halteteilbildes, das in vorab gespeicherten orthodontischen Halteteilinformationen enthalten ist, und Erzeugen eines Korrekturbildes für das orale Bild auf der Grundlage des Zahnbildes und des übereinstimmenden orthodontischen Halteteilbildes.

9. Korrekturbilderzeugungsverfahren nach Anspruch 8, umfassend des Weiteren das Bereitstellen des durch eine Bildkorrekturvorrichtung erzeugten Korrekturbildes an einen Benutzer.

10. Korrekturbilderzeugungsverfahren nach Anspruch 9, wobei das Bereitstellen des Korrekturbildes an den Benutzer des Weiteren das Bereitstellen von Halteteilnutpositionsinformationen umfasst, indem eine Position einer Halteteilnut in dem Korrekturbild auf der Grundlage der übereinstimmenden orthodontischen Halteteilinformationen berechnet wird.

11. Korrekturbilderzeugungsverfahren nach Anspruch 8, wobei das Erzeugen des Zahnbildes und des Bildes des an den mehreren Zähnen angebrachten Halteteils des Weiteren umfasst:
Erzeugen eines Bildes jedes Zahns in der Mundhöhle auf der Grundlage des oralen Bildes;
Erzeugen eines Bildes eines an jedem der Zähne angebrachten Halteteils; und
Erzeugen von Positionsinformationen des Bildes des an jedem Zahn angebrachten Halteteils auf der Grundlage einer Klebefläche in dem Bild des angebrachten Halteteils.

12. Korrekturbilderzeugungsverfahren nach Anspruch 11, wobei das Erzeugen des Korrekturbildes des Weiteren umfasst:
Vergleichen des Bildes des angebrachten Halteteils mit dem vorab gespeicherten orthodontischen Halteteilbild; und
Extrahieren eines orthodontischen Halteteilbildes, das mit dem Bild des angebrachten Halteteils in dem vorab gespeicherten orthodontischen Halteteilbild übereinstimmt.

13. Korrekturbilderzeugungsverfahren nach Anspruch 12, wobei das Erzeugen des Korrekturbildes des Weiteren das Platzieren des übereinstimmenden orthodontischen Halteteilbildes im selben Abschnitt wie das Bild des an jedem Zahn angebrachten Halteteils auf der Grundlage der Positionsinformationen des Bildes des angebrachten Halteteils umfasst.

## Revendications

1. Dispositif informatique (12) qui comporte un ou plusieurs processeurs (14), et une mémoire configurée pour stocker un ou plusieurs programmes exécutés par le ou les processeurs, le dispositif informatique (12) comprenant :
une partie d'acquisition d'image (110) configurée pour acquérir une image orale d'un intérieur de la cavité buccale d'un patient cible à l'aide d'un scanner 3D intra-oral ;
un analyseur d'image (130) configuré pour générer une image de dent et une image d'un boîtier fixé à une pluralité de dents au moyen de l'image orale obtenue ; et
un correcteur d'image (140) configuré pour faire correspondre l'image du boîtier fixé et une image de boîtier orthodontique incluse dans des informations de boîtier orthodontique pré-enregistrées et pour générer une image de correction pour l'image orale sur la base de l'image de dent et de l'image de boîtier orthodontique correspondante.

2. Dispositif informatique selon la revendication 1, comprenant en outre un fournisseur configuré pour fournir à un utilisateur l'image de correction générée par le correcteur d'image.

3. Dispositif informatique selon la revendication 2, dans lequel le fournisseur fournit des informations de position de rainure de boîtier en calculant une position d'une rainure de boîtier dans l'image de correction sur la base des informations de boîtier orthodontique correspondantes.

4. Dispositif informatique selon la revendication 1, dans lequel l'analyseur d'images génère une image de chaque dent dans la cavité buccale sur la base de l'image orale, génère une image d'un boîtier fixé à chaque dent, et génère des informations de position de l'image du boîtier fixé à chaque dent sur la base d'une surface adhésive dans l'image du boîtier fixé.

5. Dispositif informatique selon la revendication 4, dans lequel le correcteur d'image compare l'image du boîtier fixé avec l'image de boîtier orthodontique pré-enregistrée et extrait une image de boîtier orthodontique qui correspond à l'image du boîtier fixé dans l'image de boîtier orthodontique pré-enregistrée.

6. Dispositif informatique selon la revendication 5, dans lequel le correcteur d'image place l'image de boîtier orthodontique correspondante dans la même partie que l'image du boîtier fixé à chaque dent sur la base des informations de position de l'image du boîtier fixé.

7. Dispositif informatique selon la revendication 6, dans lequel le correcteur d'image met en correspondance l'image de boîtier orthodontique correspondante et l'image du boîtier fixé à l'aide d'un algorithme itératif du point le plus proche (Itérative Closest Point, ICP).

8. Procédé de génération d'image de correction pour un traitement orthodontique qui est effectué dans un dispositif informatique (12) comportant un ou plusieurs processeurs (14), et une mémoire pour stocker un ou plusieurs programmes exécutés par le ou les processeurs, le procédé comprenant :
l'obtention (S502) d'une image orale d'un intérieur de la cavité buccale d'un patient cible à l'aide d'un scanner 3D intra-oral ;
la génération (S504) d'une image de dent et d'une image d'un boîtier fixé à une pluralité de dents au moyen de l'image orale obtenue ; et
la mise en correspondance (S506) de l'image du boîtier fixé et d'une image de boîtier orthodontique incluse dans des informations de boîtier orthodontique pré-enregistrées et la génération d'une image de correction pour l'image orale sur la base de l'image de dent et de l'image de boîtier orthodontique correspondante.

9. Procédé de génération d'image de correction selon la revendication 8, comprenant en outre la fourniture de l'image de correction générée par un correcteur d'image à un utilisateur.

10. Procédé de génération d'image de correction selon la revendication 9, dans lequel la fourniture de l'image de correction à l'utilisateur comporte en outre la fourniture d'informations de position de rainure de boîtier en calculant une position d'une rainure de boîtier dans l'image de correction sur la base des informations de boîtier orthodontique correspondantes.

11. Procédé de génération d'image de correction selon la revendication 8, dans lequel la génération de l'image de dent et de l'image du boîtier fixé à la pluralité de dents comporte en outre :
la génération d'une image de chaque dent dans la cavité buccale sur la base de l'image orale ;
la génération d'une image d'un boîtier fixé à chacune des dents ; et
la génération d'informations de position de l'image du boîtier fixé à chaque dent sur la base d'une surface adhésive dans l'image du boîtier fixé.

12. Procédé de génération d'image de correction selon la revendication 11, dans lequel la génération de l'image de correction comporte en outre :
la comparaison de l'image du boîtier fixé avec l'image de boîtier orthodontique pré-enregistrée ; et
l'extraction d'une image de boîtier orthodontique qui correspond à l'image du boîtier fixé dans l'image de boîtier orthodontique pré-enregistrée.

13. Procédé de génération d'image de correction selon la revendication 12, dans lequel la génération de l'image de correction comporte en outre le placement de l'image de boîtier orthodontique correspondante dans la même partie que l'image du boîtier fixé à chaque dent sur la base des informations de position de l'image du boîtier fixé.
